# EUROPÄISCHE PATENTSCHRIFT

(11) **EP 3 104 823 B1**
(45) Veröffentlichungstag und Bekanntmachung des Hinweises auf die Patenterteilung: **22.11.2017**
(21) Anmeldenummer: 15711639.3
(22) Anmeldetag: 02.02.2015
(51) Int. Cl.: A61J 1/03

(54) **VORRICHTUNG ZUR ERKENNUNG DER ENTNAHME VON MEDIKAMENTEN**
DEVICE FOR DETECTING THE REMOVAL OF DRUGS
DISPOSITIF POUR LA DETECTION DE LA PRISE DE MEDICAMENTS

(30) Priorität: 11.02.2014 AT 501032014; 10.03.2014 AT 501732014
(43) Veröffentlichungstag der Anmeldung: 21.12.2016
(73) Patentinhaber: Seibersdorf Labor GmbH, 2444 Seibersdorf (AT); AIT Austrian Institute of Technology GmbH, 1220 Wien (AT)
(72) Erfinder: SCHMID, Gernot, 2833 Bromberg (AT); BAMMER, Manfred, 1220 Wien (AT)
(74) Vertreter: Wildhack & Jellinek
(86) Internationale Anmeldenummer: PCT/AT2015/050034
(87) Internationale Veröffentlichungsnummer: WO 2015/120498

(56) Entgegenhaltungen:
- WO-A1-89/09042
- DE-A1-102012 005 443
- US-A- 4 660 991
- US-A1- 2005 241 983

## Beschreibung

Die Erfindung betrifft eine Vorrichtung zur Erkennung der Entnahme von Medikamenten aus einem Arzneimittelblister.

Die nicht korrekte Anwendung der Arzneimittel stellt in der Praxis ein großes Problem dar. Bei richtiger Diagnose und optimalem Therapieplan kann der Therapieerfolg drastisch reduziert sein, wenn der Patient das Arzneimittel nicht richtig anwendet. Bei manchen Medikamenten, beispielsweise Blutgerinnungshemmern, kann eine falsche Anwendung sogar lebensbedrohliche Folgen haben. Die Fehlerquellen in der Praxis sind vielfältig: Patienten nehmen keine oder die falschen Medikamente, sie nehmen die richtigen Medikamente in zu kleinen oder zu hohen Dosen. Die Weltgesundheitsorganisation WHO schätzt, dass sich jeder zweite Patient nicht an Beipackzettel oder ärztliche Anweisungen hält. Experten gehen davon aus, dass in Deutschland jede vierte Krankenhauseinweisung und viele Todesfälle - alleine mehr als 40.000 pro Jahr in der Indikation "Herz-und Kreislauf" - auf eine falsche Anwendung von Medikamenten zurückzuführen sind. Die zuverlässige Erfassung der korrekten und regelmäßigen Medikamenteneinnahme durch den Patienten, einerseits aus gesundheitlichen und andererseits aus versicherungstechnischen Gründen, ist daher, zumindest für bestimmte Klassen von Medikamenten, wünschenswert.

Die gegenwärtig bestehenden Lösungsansätze zur Detektion der Tablettenentnahme aus Durchdrück-Blisterpackungen sind nur sehr eingeschränkt für den Massenmarkt tauglich und haben sich bisher nicht durchgesetzt, weil sie zu komplex in der Handhabung und in der Fertigung sind. Diese Verfahren basieren auf der Idee elektrische Leiterbahnen, Antennenstrukturen, Komponenten von Widerstandsnetzwerken, etc. durch das Herausdrücken der Tabletten zu zerstören, was durch eine an diese Strukturen angeschlossene Elektronik leicht detektierbar ist. Zu diesem Zweck müssen diese Strukturen, wie Leiterbahnen, Antennen, Widerstandsnetzwerke, etc., entweder direkt in die Verschlussfolie der Blisterpackung integriert oder nachträglich auf der Verschlussfolie aufgebracht werden, beispielsweise in Form einer Klebefolie, die die genannten Strukturen passgenau für den jeweiligen Blister enthält.

Gegenwärtig basieren kleinere klinische Studien daher auf dem Ansatz mit nachträglich auf der Unterseite von Standard Blistern passgenau aufgeklebten Folien, die die genannten Strukturen enthalten. Durch gezielt vorgesehene Sollbruchstellen in der Folien wird diese im Zuge der Tablettenentnahme gemeinsam mit der Blisterverschlussfolie durchgedrückt bzw. aufgerissen und das der jeweiligen Blistertasche bzw. Tablette zugeordnete Strukturelement, beispielsweise eine Leiterbahn, Antenne, etc, zerstört bzw. funktionsunfähig gemacht. Über eine den Strukturelementen zugeordnete Elektronik, z.B. über eine Kontaktleiste mit der Folie elektrisch verbunden, kann damit der Zeitpunkt der Tablettenentnahme und welche Tablette entnommen wurde aufgezeichnet werden. Das Hauptproblem dieser Lösungsansätze ist die Tatsache, dass die Detektion der Tablettenentnahme auf der Zerstörung der passgenau auf den Blister aufgeklebten Folie mit den Strukturelementen beruht. Es wird daher für jeden Blister einen neue Folie mit Strukturelementen benötigt, was zu einem linearen Anstieg der Kosten mit der Anzahl der benötigten Medikamentenblister führt. Der alternative Ansatz, die Strukturelemente bereits in die Blisterverschlussfolie zu integrieren wird seitens der Medikamentenhersteller als produktionstechnisch zu aufwändig, regulatorisch als zu komplex und im Hinblick auf die Kosten für die Medikamentenverpackung als nicht akzeptabel angesehen.

Eine Lösung mit einem wiederverwendbaren Detektionselement zur Überwachung der Tablettenentnahme würde sich daher nicht nur bei großen Studien bzw. bei der Massenanwendung in der Routine sehr schnell amortisieren, sondern würde auch die Herstellung hochspezialisierter Blister obsolet machen.

US 2005/241983 offenbart ein kindersicheres Medikamentenpaket. Es ist daher die Aufgabe der Erfindung, die Detektion der Tablettenentnahme zu vereinfachen und eine Vorrichtung zur Verfügung zu stellen, die die Detektion auch dann ermöglicht, wenn der Tablettenhersteller eine solche Möglichkeit nicht vorgesehen hat. Die Erfindung löst diese Aufgabe bei einer Vorrichtung der eingangs genannten Art mit den Merkmalen des Patentanspruchs 1.

Bei einer Vorrichtung zur Erkennung der Entnahme von Medikamenten aus einem Arzneimittelblister, umfassend einen Grundkörper zur Aufnahme des Arzneimittelblisters mit einer Grundfläche, die zur Anlage an die die Taschen des Blisters verschließende elektrisch leitfähige, insbesondere metallische, Folie ausgebildet ist, ist vorgesehen, dass der Grundkörper im Bereich der Taschen des Blisters Löcher aufweist, die für den Durchtritt der in den Taschen des Blisters befindlichen Medikamente ausgebildet sind,
- wobei jedes Loch jeweils im Bereich einer der Taschen angeordnet ist,
- wobei im Bereich der Löcher jeweils eine Sendespule und mindestens zwei Empfangsspulen angeordnet sind, die das jeweilige Loch umschlingen, und
- wobei die Empfangsspulen hinsichtlich der Sendespule einander zugeordnet und derart angeordnet sind, dass für den Fall, dass die im Bereich des jeweiligen Loches dem Grundkörper anliegende Folie unversehrt, insbesondere frei von Rissen ist, die Differenz der in den Empfangsspulen zufolge eines elektrischen Stromes in der Sendespule induzierten Spannungen unterhalb eines vorgegebenen Schwellwertes liegt. Durch diese Maßnahme wird eine einfache und leicht durchführbare Detektion der Öffnung einer Tasche eines Arzneimittelblisters ermöglicht.

Eine vorteilhafte Umsetzung einer automatisierten Detektion kann erzielt werden, indem eine Detektoreinheit, die die Sendespule aktiviert und die Spannungen an den Empfangsspulen misst, und die die Differenz zwischen den Spannungen an den Empfangsspulen ermittelt und für den Fall, dass die Differenz der beiden Spannungen einen vorgegebenen Schwellenwert übersteigt, eine Meldung abgibt, die das Vorliegen eines Risses in der die jeweilige Tasche verschließenden Metallfolie indiziert.

Um die Entnahmeinformationen für weitere Verarbeitung zur Verfügung zu haben, kann vorgesehen sein, dass eine Aufzeichnungseinheit, die die Detektoreinheit in vorgegebenen Zeitabständen aktiviert und das Vorhandensein von Rissen in den die Taschen des Blisters verschließenden Folien ermittelt und die diesbezügliche Information in einem Speicher abspeichert und für weitere Abfragen zur Verfügung hält.

Ein einfacher Datenaustausch wird gewährleistet, indem
- ein Kurzstrecken-Funkmodul, umfassend eine Antenne sowie einen Kommunikationscontroller, an die Detektoreinheit angeschlossen ist sowie
- ein Speicher, in den bei Erkennung eines Risses in der Folie eine diesbezügliche Meldung, insbesondere zusätzlich versehen mit einem Zeitstempel, abgespeichert wird, vorgesehen ist, wobei die Detektoreinheit im Speicher abgelegte Informationen zu einem externen Datenkommunikationsgerät übertragen kann.

Ein vorteilhafter Datenaustausch über RFID/NFC wird möglich, wenn das Kurzstreckenfunkmodul ein RFID- oder NFC-Transponder, umfassend eine Transponderantenne sowie einen Kommunikationscontroller, ist.

Hierbei ist es vorteilhaft, zur einfachen und störungsfreien Übertragung an ein externes Datenkommunikationsgerät vorzusehen, dass die Transponderantenne zumindest teilweise entlang der äußeren Begrenzung des Grundkörpers der Vorrichtung verläuft.

Alternativ kann das Kurzstreckenfunkmodul auch auf Basis eines Bluetooth-Standards arbeiten, wobei es eine Antenne sowie einen Kommunikationscontroller aufweist.

Eine vorteilhafte Auswertung einer Tasche eines Arzneimittelblisters sieht vor, dass eine Anregungseinheit vorgesehen ist, die an die Sendespule angeschlossen ist und zwei Messeinheiten vorgesehen sind, die an die Empfangsspulen angeschlossen sind, und die Detektoreinheit eine Steuereinheit aufweist, die die Anregungseinheit zur Anregung der Sendespulen ansteuert und die Messeinheiten zur Messung der an den Empfangsspulen anliegenden Induktionsspannungen ansteuert, die Differenz der ermittelten Induktionsspannungen ermittelt und für den Fall, dass der Betrag Differenz einen vorgegebenen Schwellenwert übersteigt, ein Signal abgibt.

Eine einfache Auswertung einer Vielzahl von Taschen eines Arzneimittelblisters sieht vor, dass an die Detektoreinheit ein Multiplexer für die Auswahl einer Gruppe umfassend jeweils einander zugeordnete Sende- und Empfangsspulen angeschlossen ist, wobei der Multiplexer einen gemeinsamen Eingang zur Ansteuerung der jeweiligen Sendeantenne sowie zwei gemeinsame Ausgänge zum Erhalt der von den Empfangsspulen erhaltenen Induktionsspannungen aufweist, wobei der gemeinsame Eingang an die Anregungseinheit und die gemeinsamen Ausgänge jeweils an eine der Messeinheiten angeschlossen sind, wobei der Multiplexer Gruppen umfassend jeweils zwei Multiplex-Eingänge und einen Multiplex-Ausgang aufweist, die gemeinsam adressierbar sind und jeweils mit den einander zugeordneten und im Bereich desselben Lochs angeordneten Sende- und Empfangsantennen verbunden sind.

Eine besonders genaue Detektion bei einer einfachen Bauform wird erzielt, indem die Empfangsspulen symmetrisch bezüglich der Löcher und bezüglich der Sendespule angeordnet sind.

Eine einfache Bauform sieht vor, dass die Detektoreinheit und das Kurzstreckenfunkmodul in einem separaten Gehäuse untergebracht sind und die Detektoreinheit über zerstörungsfrei trennbare elektrische Kontakte mit den am oder im Grundkörper angeordneten Sendeantennen und Empfangsantennen elektrisch verbunden ist.

Besonders vorteilhaft ist weiters eine Anordnung umfassend eine erfindungsgemäße Vorrichtung sowie einen Arzneimittelblister mit einer Anzahl von an den Löchern anliegenden und jeweils ein Medikament enthaltenden Taschen und einer die Taschen, verschließenden Folie, die an der Grundfläche anliegt, wobei jedem Loch jeweils eine Gruppe umfassend eine Sendespule und zumindest zwei Empfangsspulen gegenüberliegt.

Eine bevorzugte Ausführungsform der Erfindung wird anhand der folgenden Zeichnungsfiguren näher dargestellt.

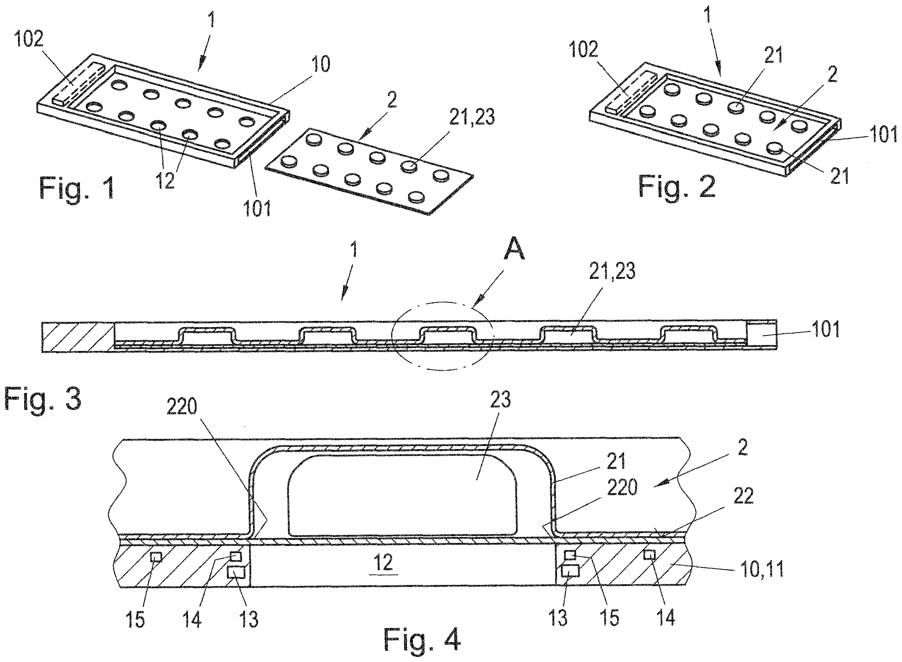

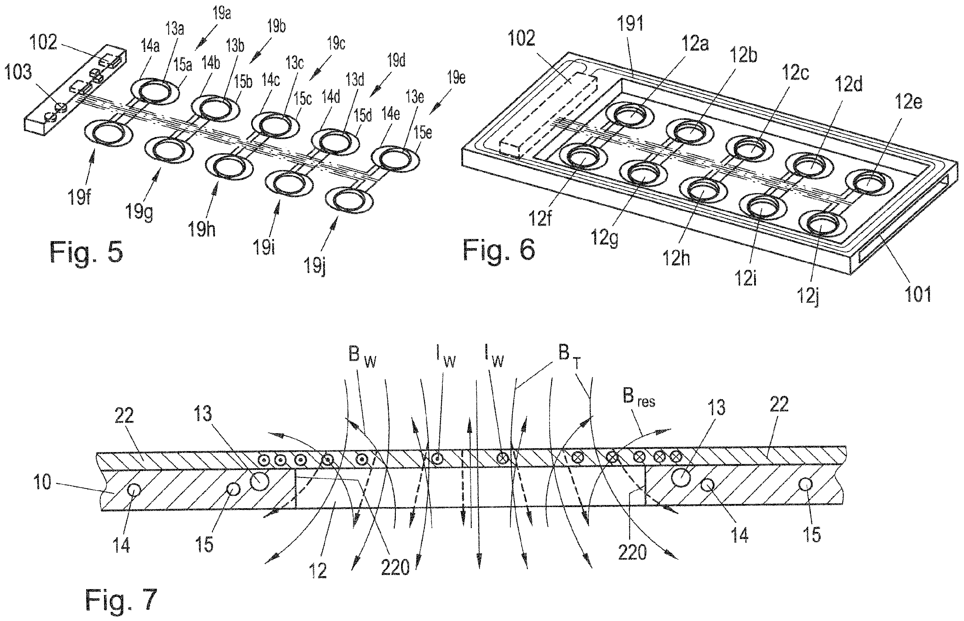

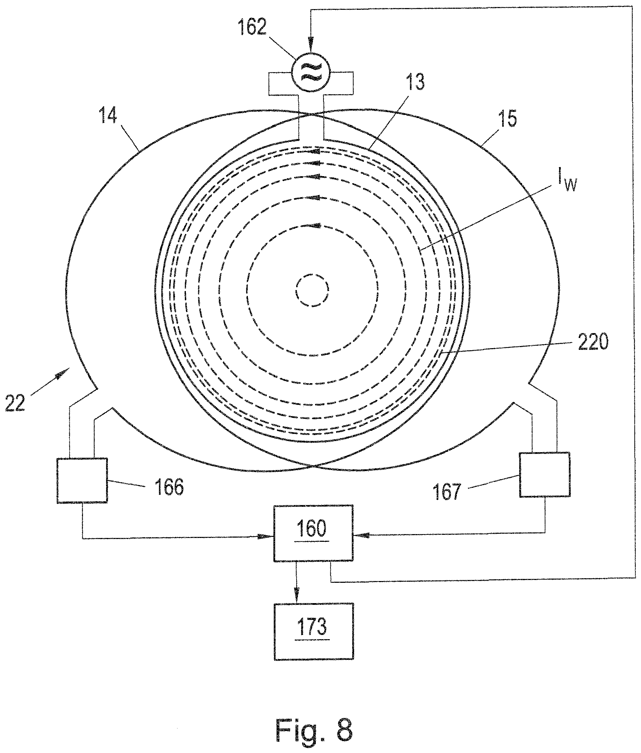

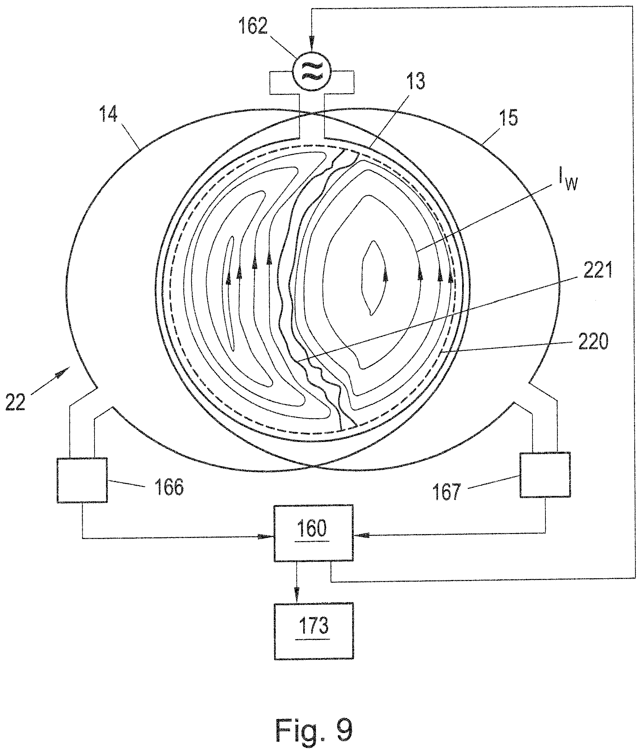

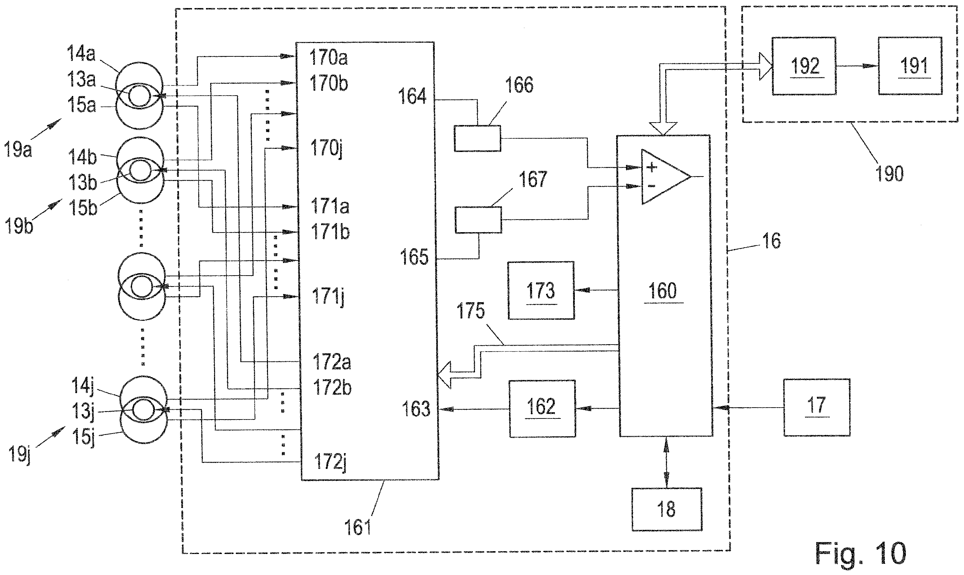

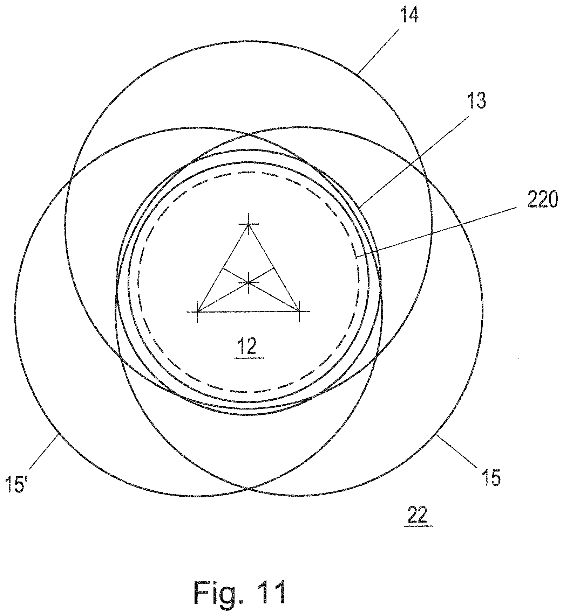

Fig. 1 zeigt eine Ausführungsform einer Vorrichtung zur Erkennung der Entnahme von Medikamenten sowie einen Arzneimittelblister. Fig. 2 zeigt die in Fig. 1 dargestellte Vorrichtung, wobei der Arzneimittelblister in die Vorrichtung eingeschoben ist. Fig. 3 zeigt die in Fig. 2 dargestellte Kombination umfassend die Vorrichtung sowie den Arzneimittelblister im Querschnitt. Fig. 4 zeigt ein Detail aus Fig. 3. Fig. 5 zeigt eine Schaltung sowie eine Anordnung von Sende- und Empfangsspulen. Fig. 6 zeigt die Einbettung der in Fig. 5 dargestellten Sende- und Empfangsspulen in der Vorrichtung zur Erkennung der Entnahme von Medikamenten. Fig. 7 zeigt die Feldverhältnisse im Bereich einer Tasche des Medikamentenbehälters in Schnittdarstellung. Fig. 8 zeigt im Detail die Feldverhältnisse im Bereich einer ungeöffneten und unversehrten Arzneimitteltasche sowie die Ansteuerung und das Auslesen der Sende- und Empfangsspulen. Fig. 9 zeigt im Detail die Feldverhältnisse im Bereich einer geöffneten und aufgerissenen Arzneimitteltasche sowie die Ansteuerung und das Auslesen der Sende- und Empfangsspulen. Fig. 10 zeigt schematisch die elektronische Messung bzw. Erkennung der Entnahme von Medikamenten. Fig. 11 zeigt eine alternative Spulenanordnung mit einer Sendespule und drei Empfangsspulen.

In **Fig. 1** ist eine erste Ausführungsform einer erfindungsgemäßen Vorrichtung zur Erkennung der Entnahme von Medikamenten 23 aus einem Arzneimittelblister 2 dargestellt. Die Vorrichtung 1 verfügt über einen Grundkörper 10 mit einer Öffnung 101 zum Einschieben des Arzneimittelblisters 2 in den Grundkörper 10. An der Position, an der sich die Medikamente 23 enthaltenden Taschen 21 des Blisters 2 befinden weist der Grundkörper 10 der Vorrichtung 1 jeweils Löcher 12 auf. Die Taschen 21 liegen somit den Löchern 12 unmittelbar gegenüber, sodass die in den Taschen 21 des Blisters 2 befindlichen Medikamente 23 aus den Taschen 21 durch die Löcher 12 hindurch aus dem Blister 2 bzw. aus der Vorrichtung 1 entnommen werden können.

**Fig. 2** zeigt den durch die Öffnung 101 hindurch geschobenen Arzneimittelblister 2.

In **Fig. 3** ist die Kombination aus der Vorrichtung zur Erkennung der Entnahme von Medikamenten 23 mit dem Arzneimittelblister 2 in Seitenansicht dargestellt. **Fig. 4** zeigt das Detail A aus **Fig. 3****.** Der Bereich der übrigen Taschen ist dabei ebenso wie der im Detail A dargestellte Bereich rund um die Tasche 21 ausgebildet. Die Tasche 21 stellt eine Ausbuchtung des Arzneimittelblisters 2 dar, in dem sich das zu entnehmende Medikament 23, das im vorliegenden Fall als Tablette 23 ausgebildet ist, befindet. Die Tasche 21 ist an der ebenen Fortsetzung des Körpers des Arzneimittelblisters 2 mit einer elektrisch leitfähigen Folie 22 überzogen, die die Tasche 21 verschließt, wobei die Folie 22 zumindest eine flächenhaft durchgehende, elektrisch leitfähige Schicht enthält oder aus dieser besteht. Im Bereich der Tasche befinden sich drei Spulen, nämlich eine Sendespule 13 sowie zwei Empfangsspulen 14, 15.

In **Fig. 5** sind die elektronischen und elektrischen Komponenten der Vorrichtung dargestellt. Die **Fig. 5** zeigt insgesamt zehn Gruppen 19a...19j von einander zugeordneten Sendespulen 13a...13j und Empfangsspulen 14a...14j, 15a...15j. Jede Gruppe 19a...19j stellt das Öffnen jeweils einer der Taschen 21 des Blisters 2 fest. In Fig. 5 ist auch eine elektronische Schaltung 102 dargestellt, die eine Steuereinheit 160 bzw. Detektoreinheit 16 (**Fig. 10**) sowie eine Spannungsversorgung 103 realisiert.

**Fig. 6** zeigt die Vorrichtung mit ihrem Gehäuse, in den die in **Fig. 5** dargestellten Sende- und Empfangsspulen 13, 14, 15 angeordnet, insbesondere eingegossen oder aufgedruckt sind. Weiters ist in **Fig. 6** eine zusätzliche Sendeantenne 191 dargestellt, mittels der die im Zusammenhang mit der Medikamentenentnahme ermittelten Daten an ein externes Datenkommunikationsgerät übertragen werden können. Im vorliegenden Ausführungsbeispiel verläuft die Sendeantenne 191 entlang der äußeren Begrenzung des Grundkörpers 10 der Vorrichtung 1.

In dieser Konfiguration und bei unversehrter Verschlussfolie 22 verteilen sich, wie in **Fig. 8** dargestellt, die aufgrund des von der Sendeantenne 13 erzeugten Magnetfeldes in der elektrisch leitfähigen Schicht der Verschlussfolie induzierten Wirbelströme I_{w} kreisförmig im Bereich der Tasche 21. Aufgrund der jeweiligen Flussverkettungen entsteht in den beiden Empfangsspulen 14, 15 jeweils eine Induktionsspannung, die von den Messgeräten 166, 167 ermittelt und an eine Steuereinheit 160 weitergeleitet wird.

In **Fig. 7** ist die Feld- und Stromverteilung bei ungeöffneter Tasche 21 und unversehrter Folie 22 im Bereich der Tasche dargestellt. Fig. 7 zeigt im Schnitt die Anordnung der Sendespule 13 sowie der beiden Empfangsspulen 14, 15, die das Loch 12 des Grundkörpers 10 der Vorrichtung 1 umschlingen. Oberhalb des Lochs 12 ist die, die Tasche 21 verschließende Folie 22 dargestellt. Aufgrund der Anregung in der Sendespule 13, würde bei Abwesenheit der Folie 22 im Bereich des Lochs 12 ein ungestörtes magnetisches Feld B_{T} entstehen. Aufgrund der in der Folie 22 induzierten Wirbelströme I_{w} wird jedoch ein Gegenfeld B_{w} erstellt, das in Überlagerung mit dem Anregungsfeld B_{T} ein resultierendes Feld Bᵣₑₛ ergibt, das gegenüber dem ursprünglich anregenden Feld B_{T} wesentlich geschwächt ist.

Reißt die die Tasche 21 verschließende Folie 22 auf, so verteilen sich die in der Folie 22 unterhalb der Tasche 21 induzierten Wirbelströme unregelmäßig.

**Fig. 9** ist eine Stromkonfiguration von Wirbelströmen I_{w} dargestellt, wobei die Folie 22 im Bereich der Tasche 21 gerissen ist. Aufgrund der unterschiedlichen Ausrichtung und Größe der durch den Riss entstandenen Reste der Folie 22 werden in den beiden Empfangsspulen 14, 15 unterschiedliche Spannungen induziert, die über die Spannungsmessgeräte 166, 167 wahrgenommen werden. Die Steuereinheit 160 detektiert in diesem Fall eine Spannungsdifferenz und gibt dementsprechend eine Erkennungsmeldung 173 aus, die das Erkennen des Aufreißens oder der Entnahme des Medikamentes 23 indiziert. Besonders große und daher leicht detektierbare Spannungsdifferenzen entstehen durch die Tatsache, dass sich in der Praxis die nach dem Aufreißen der Folie 22 entstehenden Folienreste unterhalb der Tasche 21 zumindest geringfügig bzw. teilweise aus der Ebene der Folie 22 herausdrehen. Dadurch fließen die in diesen Folienresten induzierten Wirbelströme auch nicht mehr in der Ebene der Folie 22, was zu einer komplexen dreidimensionalen Verteilung des resultierenden Magnetfeldes Bᵣₑₛ führt, die deutlich von der Magnetfeldverteilung bei unversehrter Verschlussfolie 22 abweicht.

In **Fig. 10** ist eine Detektoreinheit 16 dargestellt, mit der die Entnahme einer Vielzahl von Medikamenten 23 aus Taschen im selben Arzneimittelblister 2 detektiert werden kann. Die Detektoreinheit 16 verfügt dabei über die Steuereinheit 160 und einen Multiplexer 161 für die Auswahl der jeweiligen Gruppe 19a...19j, umfassend jeweils einander zugeordnete Sende- und Empfangsspulen 13a...13j, 14a...14j und 15a...15j. Der Multiplexer weist einen gemeinsamen Eingang 163 zur Ansteuerung der jeweiligen Sendeantenne 13 auf. Diesem Anschluss ist der Spannungsgenerator 162 zugeschaltet, der von der Steuereinheit 160 gesteuert ist. Weiters weist der Multiplexer 161 zwei gemeinsame Ausgänge 164, 165 auf, die jeweils einem der Spannungsmessgeräte 166, 167 zugeordnet sind. Die Ergebnisse der Spannungsmessung werden von den Spannungsmessgeräten 166, 167 an die Steuereinheit 160 übermittelt. Die Steuereinheit 160 legt weiters über den Multiplexsteuerausgang 175 die jeweilige Gruppe von Sende- und Empfangsspulen 13a, 14a, 15a...13j, 14j, 15j fest, die jeweils angesprochen werden, um zu ermitteln, ob das jeweilige Medikament 23 aus der jeweils zugeordneten Tasche 21a...21j entnommen wurde. Der Multiplexer 161 weist gruppenumfassend jeweils zwei Multiplexeingänge 170a, 171a...170j, 171j und einen Multiplexausgang 172a...172j auf, wobei jede der Gruppen jeweils separat adressierbar ist. Die Multiplexeingänge und Multiplexausgänge, die einander in Gruppen 19a...19j zugeordnet sind, sind jeweils mit einander zugeordneten und gruppierten und im Bereich desselben Lochs 12 angeordneten Sende- und Empfangsantennen 13a...13j, 14a...14j, 15a... 15j verbunden.

Um zu detektieren, ob die im Bereich des jeweiligen Loches 12 dem Grundkörper 10 anliegende metallische Folie 22 unversehrt, insbesondere frei von Rissen ist, wird die Differenz der in den Empfangsspulen 14,15 zufolge eines elektrischen Stromes in der Sendespule 13 induzierte Spannung gemessen. Liegt diese unterhalb eines vorgegebenen Schwellwertes, so kann die Folie 22 im Bereich des jeweiligen Loches 12 als unversehrt angesehen werden.

Die Detektoreinheit 16 misst die beiden an den Empfangsspulen 14, 15 anliegenden Spannungen und bestimmt die Differenz zwischen den Spannungen an den Empfangsspulen 14, 15. Für den Fall, dass die Differenz der beiden Spannungen einen vorgegebenen Schwellenwert übersteigt, gibt sie eine Meldung ab, die das Vorliegen eines Risses in der die jeweilige Tasche 21 verschließenden Metallfolie 22 indiziert.

Um eine Kommunikation mit einem externen Datenkommunikationsgerät zu ermöglichen, ist die Steuereinheit 160 mit einem Kurzstrecken-Funkmodul 190 verbunden, das eine Antenne 191 sowie einen Kommunikationscontroller 192 umfasst. Bei diesem Kurzstrecken-Funkmodul kann es sich sowohl um einen RFID- oder NFC-Transponder als auch um eine andere drahtlose Kurzstreckenkommunikationstechnik, wie z.B. Bluetooth handeln. Weiters ist die Steuereinheit 160 an einen Speicher 18 angeschlossen, wobei die Steuereinheit 160 bei Detektion der Entnahme eines Medikamentes 23 aus einer der Taschen jeweils eine diesbezügliche Meldung im Speicher 18 abspeichert und für einen Abruf seitens eines externen Datenkommunikationsgerätes zur Verfügung hält.

Die Detektoreinheit 16 und das Kurzstreckenfunkmodul 190 können insbesondere auch in einem separaten Gehäuse untergebracht sein und die Detektoreinheit 16 ist über zerstörungsfrei trennbare elektrische Kontakte mit den am oder im Grundkörper 10 angeordneten Sendeantennen 13 und Empfangsantennen 14,15 elektrisch verbunden. Weiters ist in **Fig. 10** eine Aufzeichnungseinheit 17 dargestellt, die die Aufzeichnung der Entnahme von Medikamenten in vorgegebenen Zeitabständen auslöst. Die aufgenommenen Werte bzw. Meldungen, die die Entnahme von Medikamenten repräsentieren, werden im Speicher 18 abgespeichert.

In **Fig. 11** ist eine alternative Ausführungsform der Anordnung von Sende- und Empfangsspulen dargestellt. Diese besondere Anordnung umfasst eine Sendespule 13 sowie drei Empfangsspulen 14, 15, 15', die allesamt kreisförmig ausgebildet sind. Die Mittelpunkte, der Empfangsspulen 14, 15, 15' befinden sich auf einem gleichseitigen Dreieck, in dessen Schwerpunkt der Mittelpunkt der Sendespule 13 liegt. Weiters ist die Kante 220 der Tasche 21 an der die Folie 22 anschließt konzentrisch ausgebildet zu der Sendespule 13.

## Patentansprüche

1. Vorrichtung (1) zur Erkennung der Entnahme von Medikamenten (23) aus einem Arzneimittelblister (2), umfassend einen Grundkörper (10) zur Aufnahme des Arzneimittelblisters (2) mit einer Grundfläche (11), die zur Anlage an die die Taschen (21) des Blisters (2) verschließende elektrisch leitfähige, insbesondere metallische, Folie (22) ausgebildet ist, wobei der Grundkörper (10) im Bereich der Taschen (21) des Blisters (2) Löcher (12) aufweist, die für den Durchtritt der in den Taschen (21) des Blisters (2) befindlichen Medikamente (23) ausgebildet sind,
- wobei jedes Loch (12) jeweils im Bereich einer der Taschen (21) angeordnet ist, **dadurch gekennzeichnet, dass**
- im Bereich der Löcher (12) jeweils eine Sendespule (13) und mindestens zwei Empfangsspulen (14, 15) angeordnet sind, die das jeweilige Loch (12) umschlingen, und
- dass die Empfangsspulen (14, 15) hinsichtlich der Sendespule (12) einander zugeordnet und derart angeordnet sind, dass für den Fall, dass die im Bereich des jeweiligen Loches (12) dem Grundkörper (10) anliegende Folie (22) unversehrt, insbesondere frei von Rissen ist, die Differenz der in den Empfangsspulen (14,15) zufolge eines elektrischen Stromes in der Sendespule (13) induzierten Spannungen unterhalb eines vorgegebenen Schwellwertes liegt.

2. Vorrichtung nach Anspruch 1, **gekennzeichnet durch** eine Detektoreinheit (16), die die Sendespule (13) aktiviert und die Spannungen an den Empfangsspulen (14, 15) misst, und die die Differenz zwischen den Spannungen an den Empfangsspulen (14, 15) ermittelt und für den Fall, dass die Differenz der beiden Spannungen einen vorgegebenen Schwellenwert übersteigt, eine Meldung abgibt, die das Vorliegen eines Risses in der die jeweilige Tasche (21) verschließenden Metallfolie (22) indiziert.

3. Vorrichtung (1) nach Anspruch 1 oder 2 **gekennzeichnet durch** eine Aufzeichnungseinheit (17), die die Detektoreinheit (16) in vorgegebenen Zeitabständen aktiviert und das Vorhandensein von Rissen in den die Taschen (21) des Blisters (2) verschließenden Folien (22) ermittelt und die diesbezügliche Information in einem Speicher (18) abspeichert und für weitere Abfragen zur Verfügung hält.

4. Vorrichtung (1) nach einem der vorangehenden Ansprüche, **gekennzeichnet durch**
- ein Kurzstrecken-Funkmodul (190), umfassend eine Antenne (191) sowie einen Kommunikationscontroller (192), das an die Detektoreinheit (16) angeschlossen ist sowie
- einen Speicher (18), in den bei Erkennung eines Risses in der Folie (22) eine diesbezügliche Meldung, insbesondere zusätzlich versehen mit einem Zeitstempel, abgespeichert wird, wobei die Detektoreinheit (16) im Speicher (18) abgelegte Informationen zu einem externen Datenkommunikationsgerät übertragen kann.

5. Vorrichtung (1) nach Anspruch 4, **dadurch gekennzeichnet, dass** das Kurzstreckenfunkmodul (190) ein RFID- oder NFC-Transponder, umfassend eine Transponderantenne (191) sowie einen Kommunikationscontroller (192), ist.

6. Vorrichtung (1) nach Anspruch 5, **dadurch gekennzeichnet, dass** die Transponderantenne (191) zumindest teilweise entlang der äußeren Begrenzung des Grundkörpers (10) der Vorrichtung (1) verläuft.

7. Vorrichtung (1) nach Anspruch 4, **dadurch gekennzeichnet, dass** das Kurzstreckenfunkmodul (190), umfassend eine Antenne (191) sowie einen Kommunikationscontroller (192), auf Basis eines Bluetooth-Standards arbeitet.

8. Vorrichtung (1) nach einem der vorangehenden Ansprüche, **dadurch gekennzeichnet, dass** eine Anregungseinheit (162) vorgesehen ist, die an die Sendespule (13) angeschlossen ist und zwei Messeinheiten (166, 167) vorgesehen sind, die an die Empfangsspulen (14, 15) angeschlossen sind, und die Detektoreinheit (16) eine Steuereinheit (160) aufweist, die die Anregungseinheit (162) zur Anregung der Sendespulen (13) ansteuert und die Messeinheiten (166, 167) zur Messung der an den Empfangsspulen (14, 15) anliegenden Induktionsspannungen ansteuert, die Differenz der ermittelten Induktionsspannungen ermittelt und für den Fall, dass der Betrag Differenz einen vorgegebenen Schwellenwert übersteigt, ein Signal abgibt.

9. Vorrichtung (1) nach einem der vorangehenden Ansprüche, **dadurch gekennzeichnet, dass** an die Detektoreinheit (16) ein Multiplexer (161) für die Auswahl einer Gruppe (19) umfassend jeweils einander zugeordnete Sende- und Empfangsspulen (13, 14, 15) angeschlossen ist, wobei der Multiplexer (161) einen gemeinsamen Eingang (163) zur Ansteuerung der jeweiligen Sendeantenne (13) sowie zwei gemeinsame Ausgänge (164, 165) zum Erhalt der von den Empfangsspulen erhaltenen Induktionsspannungen aufweist, wobei der gemeinsame Eingang (163) an die Anregungseinheit (162) und die gemeinsamen Ausgänge (164, 165) jeweils an eine der Messeinheiten (166, 167) angeschlossen sind,
wobei der Multiplexer (161) Gruppen umfassend jeweils zwei Multiplex-Eingänge (170a, 171a;...170j, 171j) und einen Multiplex-Ausgang (172a...172j) aufweist, die gemeinsam adressierbar sind und jeweils mit den einander zugeordneten und im Bereich desselben Lochs (12) angeordneten Sende- und Empfangsantennen (13, 14, 15) verbunden sind.

10. Vorrichtung (1) nach einem der vorangehenden Ansprüche, **dadurch gekennzeichnet, dass** die Empfangsspulen (14,15) symmetrisch bezüglich der Löcher (12) und bezüglich der Sendespule (13) angeordnet sind.

11. Vorrichtung (1) nach einem der vorangehenden Ansprüche, **dadurch gekennzeichnet, dass** die Detektoreinheit (16) und das Kurzstreckenfunkmodul (190) in einem separaten Gehäuse untergebracht sind und die Detektoreinheit (16) über zerstörungsfrei trennbare elektrische Kontakte mit den am oder im Grundkörper (10) angeordneten Sendeantennen (13) und Empfangsantennen (14,15) elektrisch verbunden ist.

12. Anordnung umfassend eine Vorrichtung (1) nach einem der vorangehenden Ansprüche sowie einen Arzneimittelblister (2) mit einer Anzahl von an den Löchern (12) anliegenden und jeweils ein Medikament (23) enthaltenden Taschen (21) und einer die Taschen (21), verschließenden Folie (22), die an der Grundfläche (11) anliegt, wobei insbesondere jedem Loch (12) jeweils eine Gruppe umfassend eine Sendespule (13) und zumindest zwei Empfangsspulen (14, 15) gegenüberliegt.

## Claims

1. A device (1) for detecting the removal of medication (23) from a pharmaceutical blister pack (2), comprising a base body (10) for receiving the pharmaceutical blister pack (2) which has a base surface (11) which is configured to rest on the electrically conductive, in particular metallic, film (22) sealing the pockets (21) of the blister pack (2) wherein the base body (10) comprises holes (12) in the region of the pockets (21) of the blister pack (2) which are configured for the passage of the medication (23) located in the pockets (21) of the blister pack (2),
- wherein each hole (12) is arranged in the region of one of the pockets (21), **characterised in that**
- one transmitting coil (13) and at least two receiving coils (14, 15) are arranged in the region of the holes (12), said coils enclosing the respective holes (12) and
- **in that** the receiving coils (14, 15) are associated with each with respect to the transmitting coil (12) and arranged such that in the case where the film (22) adjacent to the base body (10) in the region of the respective hole (12) is intact, in particular free of tears, the difference in the voltages induced in the receiving coils (14, 15) as a result of an electric current in the transmitting coil (13) is below a predefined threshold value.

2. A device according to claim 1, **characterised by** a detector unit (16) which activates the transmitting coil (13) and measures the voltages on the receiving coils (14, 15) and which determines the difference between the voltages on the receiving coils (14, 15) and in the case where the difference between the two voltages exceeds a predefined threshold valve, the detector unit sends a message which indicates the presence of a tear in the metal film (22) sealing the pocket (21).

3. A device (1) according to claim 1 or 2, **characterised by** a recording unit (17) which activates the detector unit (16) at predefined time intervals and determines the presence of tears in the films (22) sealing the pockets (21) of the blister pack (2) and stores the related information in a memory (18) and keeps it available for further enquiries.

4. A device (1) according to any one of the preceding claims, **characterised by**
- a short-range radio module (190), comprising an antenna (191) as well as a communication controller (192) which is connected to the detector unit (16) as well as
- a memory (18) in which, in the event that a tear in the film (22) is detected, a related message, in particular provided with a time stamp, is stored, wherein the detector unit (16) can transfer information stored in the memory (18) to an external data communication device.

5. A device (1) according to claim 4, **characterised in that** the short-range radio module (190) is an RFID or NFC transponder comprising a transponder antenna (191) as well as a communication controller (192).

6. A device (1) according to claim 5, **characterised in that** the transponder antenna (191) runs at least partly along the outer limit of the base body (10) of the device (1).

7. A device (1) according to claim 4, **characterised in that** the short-range radio module (190), comprising an antenna (191) as well as a communication controller (192) operates on the basis of a Bluetooth standard.

8. A device (1) according to any one of the preceding claims, **characterised in that** an excitation unit (162) is provided which is connected to the transmitting coil (13) and two measuring units (166, 167) are provided which are connected to the receiving coils (14, 15) and the detector unit (16) comprises a control unit (160) which actuates the excitation unit (162) to excite the transmitting coils (13) and actuates the measuring units (166, 167) to measure the induction voltages applied to the receiving coils (14, 15) which determines the difference between the determined induction voltages and in the case where the amount of the difference exceeds a predefined threshold valve, same emits a signal.

9. A device (1) according to any one of the preceding claims, **characterised in that** a multiplexer (161) for selecting a group (19) comprising transmitting and receiving coils (13, 14, 15) each assigned to each other is connected to the detector unit (16), wherein the multiplexer (161) comprises a common input (163) to actuate the transmitting antenna (13) as well as two common outputs (164, 165) to receive the induction voltages received by the receiving coils, wherein the common input (163) is connected to the excitation unit (162) and the common outputs (164, 165) are each connected to one of the measuring units (166, 167),
wherein the multiplexer (161) comprises groups each comprising two multiplex inputs (170a, 171a;... 170j, 171j) and a multiplex output (172a... 172j) which are mutually addressable and are each connected to the transmitting and receiving antennas (13, 14, 15) assigned to each other and arranged in the region of the same hole (12).

10. A device (1) according to any one of the preceding claims, **characterised in that** the receiving coils (14, 15) are arranged symmetrically in relation to the holes (12) and in relation to the transmitting coil (13).

11. A device (1) according to any one of the preceding claims, **characterised in that** the detector unit (16) and the short-range radio module (190) are housed in a separate housing and the detector unit (16) is electrically connected to the transmitting antennas (13) and receiving antennas (14, 15) arranged on or in the base body (10) via electrical contacts that are separable in a non-destructive manner.

12. An arrangement comprising a device (1) according to any one of the preceding claims as well as a pharmaceutical blister pack (2) having a number of pockets (21) which are in contact with the holes (12) and in each case contain medication (23) and a film (22) sealing the pockets (21), which is in contact with the base surface (11), wherein in particular a group comprising one transmitting coil (13) and at least two receiving coils (14, 15) is located in each case opposite each hole (12).

## Revendications

1. Dispositif (1) destiné à détecter la prise de médicaments (23) dans une plaquette de médicaments (2), comprenant un corps de base (10) destiné à recevoir la plaquette de médicaments (2) avec une surface de base (11), qui est conçue pour s'appuyer contre la feuille (22) conductrice de l'électricité, en particulier métallique, qui ferme les alvéoles (21) de la plaquette (2), le corps de base (10) présentant, dans la région des alvéoles (21) de la plaquette (2), des trous (12), conçus pour fournir un passage aux médicaments (23) contenus dans les alvéoles (21) de la plaquette (2),
- chaque trou (12) étant disposé dans la région d'une des alvéoles (21), **caractérisé en ce que**
- dans la région de chaque trou (12) sont disposés une bobine émettrice (13) et au moins deux bobines réceptrices (14, 15), qui encerclent le trou respectif (12) et
- **en ce que** les bobines réceptrices (14, 15) sont associées l'une à l'autre par rapport à la bobine émettrice (12) et disposées de telle sorte que, pour le cas où la feuille (22) appliquée dans la région du trou respectif (12) du corps de base (10) est intacte, en particulier exempte de fissures, la différence des tensions induites dans les bobines réceptrices (14, 15) sous l'effet d'un courant électrique passant dans la bobine émettrice (13) se trouve au-dessus d'une valeur seuil prédéfinie.

2. Dispositif selon la revendication 1, **caractérisé par** une unité de détection (16), qui active la bobine émettrice (13) et qui mesure les tensions au niveau des bobines réceptrices (14, 15) et qui détermine la différence entre les tensions au niveau des bobines réceptrices (14, 15) et qui, au cas où la différence entre les deux tensions dépasse une valeur seuil prédéfinie, délivre un message, qui indique la présence d'une fissure dans la feuille métallique (22) qui ferme l'alvéole (21) respective.

3. Dispositif (1) selon la revendication 1 ou 2, **caractérisé par** une unité d'enregistrement (17), qui active l'unité de détection (16) à intervalles prédéfinis et détermine la présence de fissures dans les feuilles (22) fermant les alvéoles (21) de la plaquette (2) et qui mémorise les informations à ce sujet dans une mémoire (18) et les maintient à disposition pour des requêtes ultérieures.

4. Dispositif (1) selon une des revendications précédentes, **caractérisé par**
- un module radio à courte portée (190), comprenant une antenne (191) ainsi qu'un organe de commande de communication (192), qui est raccordé à l'unité de détection (16) ainsi que par
- une mémoire (18), dans laquelle, lors de la détection d'une fissure dans la feuille (22), un message à ce sujet, notamment comprenant en outre un horodatage, est mémorisé, l'unité de détection (16) pouvant transmettre des informations mémorisées dans la mémoire (18) à un appareil de communication de données externe.

5. Dispositif (1) selon la revendication 4, **caractérisé en ce que** le module radio à courte portée (190) est un transpondeur RFID ou NFC, comprenant une antenne de transpondeur (191), ainsi qu'un organe de commande de communication (192).

6. Dispositif (1) selon la revendication 5, **caractérisé en ce que** l'antenne de transpondeur (191) s'étend au moins partiellement le long de la limite extérieure du corps de base (10) du dispositif (1).

7. Dispositif (1) selon la revendication 4, **caractérisé en ce que** le module radio à courte portée (190), comprenant une antenne (191) ainsi qu'un organe de commande de communication (192), fonctionne sur la base d'une norme Bluetooth.

8. Dispositif (1) selon une des revendications précédentes, **caractérisé en ce qu'**une unité d'excitation (162) est prévue, raccordée à la bobine émettrice (13) et **en ce que** deux unités de mesure (166, 167) sont prévues, raccordées aux bobines réceptrices (14, 15) et **en ce que** l'unité de détection (16) présente une unité de régulation (160), qui commande l'unité d'excitation (162) pour l'excitation de la bobine émettrice (13) et les unités de mesure (166, 167) pour la mesure des tensions d'induction actives au niveau des bobines réceptrices (14, 15), détermine la différence entre les tensions d'induction déterminées et, au cas où la différence dépasse une valeur seuil prédéfinie, délivre un signal.

9. Dispositif (1) selon une des revendications précédentes, **caractérisé en ce qu'**un multiplexeur (161) pour la sélection d'un groupe (19) comprenant respectivement des bobines émettrice et réceptrices (13, 14, 15) associées est raccordé à l'unité de détection (16), le multiplexeur (161) présentant une entrée commune (163) pour le pilotage de l'antenne émettrice (13) respective ainsi que deux sorties communes (164, 165) pour la réception des tensions d'indiction reçues par les bobines réceptrices, l'entrée commune (163) étant raccordée à l'unité d'excitation (162) et les sorties communes (164, 165) étant raccordées à une des unités de mesure (166, 167) respectivement,
le multiplexer (161) présentant des groupes comprenant respectivement deux entrées de multiplexage (170a, 171a ; ... 170j, 171j) et une sortie de multiplexage (172a... 172j) qui peuvent être adressées de façon commune et étant reliées avec les antennes émettrice et réceptrice (13, 14, 15) disposées dans la région du même trou (12) et associées les unes aux autres.

10. Dispositif (1) selon une des revendications précédentes, **caractérisé en ce que** les bobines réceptrices (14, 15) sont placées de façon symétrique par rapport aux trous (12) et par rapport à la bobine émettrice (13).

11. Dispositif (1) selon une des revendications précédentes, **caractérisé en ce que** l'unité de détection (16) et le module radio à courte portée (190) sont logés dans un boîtier séparé et **en ce que** l'unité de détection (16) est reliée par voie électrique, par le biais de contacts électriques séparables sans dégradation, avec les antennes émettrice (13) et réceptrices (14, 15) disposées au niveau du corps de base (10) ou dans celui-ci.

12. Agencement comprenant un dispositif (1) selon une des revendications précédentes, ainsi qu'une plaquette de médicaments (2) avec un certain nombre d'alvéoles (21) adjacentes aux trous (12) et contenant respectivement un médicament (23) et une feuille (22) fermant les alvéoles (21), qui repose sur la surface de base (11), chaque trou (12) étant en particulier opposé à un groupe comprenant une bobine émettrice (13) et au moins deux bobines réceptrices (14, 15).
